# EUROPÄISCHE PATENTSCHRIFT

(11) **EP 2 814 534 B1**
(45) Veröffentlichungstag und Bekanntmachung des Hinweises auf die Patenterteilung: **05.07.2017**
(21) Anmeldenummer: 13705967.1
(22) Anmeldetag: 14.02.2013
(51) Int. Cl.: A61M 1/10, A61M 1/12

(54) **INTRAVASALE BLUTPUMPE**
INTRAVASCULAR BLOODPUMP
POMPE A SANG INTRAVASCULAIRE

(30) Priorität: 16.02.2012 DE 102012202411
(43) Veröffentlichungstag der Anmeldung: 24.12.2014
(73) Patentinhaber: Abiomed Europe GmbH, 52074 Aachen (DE)
(72) Erfinder: SPANIER, Gerd, 52074 Aachen (DE); KIRCHHOFF, Frank, 52074 Aachen (DE); SIESS, Thorsten, 52074 Aachen (DE); MICHELS, Dirk, 52074 Aachen (DE)
(74) Vertreter: Klunker IP Patentanwälte PartG mbB
(86) Internationale Anmeldenummer: PCT/EP2013/053001
(87) Internationale Veröffentlichungsnummer: WO 2013/120957

(56) Entgegenhaltungen:
- EP-B1- 0 961 621
- US-A- 4 944 722
- US-A- 5 211 546
- US-A1- 2007 156 006
- US-A1- 2010 174 131

## Beschreibung

Die Erfindung betrifft eine intravasale Blutpumpe zur Unterstützung des Blutkreislaufs im menschlichen oder gegebenenfalls auch tierischen Körpers. Sie wird perkutan beispielsweise in die Femoralarterie eingeführt und durch das Gefäßsystem des Körpers hindurchgeführt, um beispielsweise die Pumparbeit im Herzen zu unterstützen oder zu ersetzen. Die Erfindung betrifft gleichermaßen ein eine solche intravasale Blutpumpe umfassendes System.

Die Anforderungen an derartige Blutpumpen hinsichtlich Einsatzdauer und geringer Größe nehmen ständig zu. Die kleinsten Pumpen dieser Art haben einen Außendurchmesser von etwa 4 mm. Eine weitere Reduzierung des Außendurchmessers wird unter anderem durch die in der Pumpe zum Einsatz kommenden Maschinenelemente begrenzt, die nicht in beliebig geringen Größen zur Verfügung stehen. Darüber hinaus sind die Maschinenelemente enormen Belastungen ausgesetzt, denn aufgrund der geringen Baugröße und der doch beträchtlichen zu fördernden Volumenströme im menschlichen Blutkreislauf arbeiten diese Pumpen mit hohen Drehzahlen von mehreren 10.000 Umdrehungen pro Minute. Waren diese Art von Blutpumpen ursprünglich nur zur kurzzeitigen Herzunterstützung bestimmt, so werden sie zunehmend auch zur Langzeittherapie über mehrere Tage bis zu Wochen eingesetzt.

Aus EP 0 961 621 B1 ist eine intravasale Blutpumpe bekannt, die einen Antriebsteil, einen am proximalen Ende des Antriebsteils angeschlossenen Katheter, durch den hindurch Leitungen zur Stromversorgung des Antriebsteils verlaufen, und einen am distalen Ende des Antriebsteils befestigten Pumpenteil besitzt. Der Antriebsteil umfasst ein Motorgehäuse mit einem darin angeordneten Elektromotor, wobei die Motorwelle des Elektromotors distal aus dem Antriebsteil heraus und in den Pumpenteil hineinragt. Der Pumpenteil seinerseits umfasst ein rohrförmiges Pumpengehäuse, in dem ein Flügelrad dreht, welches auf dem aus dem Motorgehäuse herausragenden Ende der Motorwelle sitzt. Dabei ist die Motorwelle in dem Motorgehäuse in genau zwei Lagern gelagert, die maximal voneinander entfernt sind, um eine schlagfreie zentrisch genaue Führung des Flügelrads im Pumpengehäuse zu gewährleisten. Während für das Lager am proximalen Ende des Motorgehäuses in der Praxis ein Radialkugellager eingesetzt wird, ist das flügelradseitige Lager zudem als Wellendichtung ausgebildet, um zu verhindern, dass Blut in das Motorgehäuse eintritt. In der Praxis wird dem Eintritt von Blut in das Motorgehäuse desweiteren dadurch entgegengewirkt, dass eine Spülflüssigkeit durch das Motorgehäuse und das als Wellendichtung ausgebildete flügelradseitige Lager hindurch geleitet wird. Dies geschieht mit einem Spülflüssigkeitsdruck, der über dem anliegenden Blutdruck liegt.

Die intravasale Blutpumpe fördert das Blut normalerweise von distal nach proximal durch das Pumpengehäuse hindurch und am Motorgehäuse vorbei. Auch eine umgekehrte Förderung ist möglich. In beiden Fällen erzeugt das Flügelrad bei der Blutförderung Axialkräfte, die über die Motorwelle auf die Lagerungen übertragen und von dem Radialkugellager aufgenommen werden.

US 2010/0174131 A1 offenbart eine Blutpumpe, die ähnlich aufgebaut ist wie die oben beschriebene Blutpumpe, wobei jedoch zur Kompensation von Axialkräften am proximalen Ende des Rotors des Elektromotors ein Axialgleitlager und ein Radial-Axial-Gleitlager vorgesehen sind. Dazu ist das proximale Ende des Rotors konusförmig ausgebildet.

Ausgehend von diesem Stand der Technik ist es die Aufgabe der vorliegenden Erfindung, Maßnahmen vorzuschlagen, wie die Baugröße derartiger intravasaler Blutpumpen weiter reduziert und ihre Lebensdauer erhöht werden kann.

Diese Aufgabe wird durch eine intravasale Blutpumpe mit den Merkmalen des Anspruchs 1 gelöst. In davon abhängigen Ansprüchen sind vorteilhafte Weiterbildungen und Ausgestaltungen der Erfindung angegeben.

Die erfindungsgemäße Blutpumpe zeichnet sich dadurch aus, dass sie mittels eines Axiallagers axial in dem Motorgehäuse gelagert ist, wobei das Axiallager ein Axialgleitlager oder kombiniertes Radial-Axial-Gleitlager ist. Die axialen Kräfte der Motorwelle brauchen somit nicht mehr von dem am proximalen Ende des Motor-gehäuses angeordneten Radialkugellager aufgenommen zu werden. Das Radialkugellager kann daher entsprechend kleiner gebaut werden oder durch ein anderes klein bauendes Radiallager, insbesondere ein Radialgleitlager, ersetzt werden. Dadurch wiederum ist es möglich, Blutpumpen mit einem weiter reduzierten Außendurchmesser zu entwickeln.

Gleichzeitig verlängert sich durch diese Maßnahme die Lebensdauer der Blutpumpe, weil das Radiallager aufgrund der verringerten axialen Kräfte, zu deren Aufnahme ein Radiallager ohnehin nicht primär bestimmt ist, entlastet ist, so dass es einem geringeren Verschleiß unterliegt.

Alternativ kann die Erfindung in vorhandene Baugrößen integriert werden, um die Lebensdauer zu erhöhen und den Komplexitätsgrad zu reduzieren.

Die auf die Motorwelle wirkenden Axialkräfte sind der Förderrichtung entgegengesetzt. Wenn die Blutpumpe dazu eingerichtet ist, alternativ in proximaler Richtung und in distaler Richtung zu fördern, so wirken auf die Motorwelle in dem einen Fall Axialkräfte in distaler Richtung und in dem anderen Fall in proximaler Richtung. Bei einer solchen Blutpumpe sind dementsprechend zwei Axialgleitlager oder Axial-Radial-Gleitlager in dem Motorgehäuse zur axialen Lagerung der Motorwelle vorzusehen. Das Axialgleitlager kann in einfacher Weise durch eine auf der Motorwelle angeordnete Scheibe gebildet werden, die sich gegen eine umlaufende Schulter des Motorgehäuses abstützt. Im Falle eines Axial-Radial-Gleitlagers besitzt die Scheibe eine konvexe oder konkave, insbesondere sphärische, Lagerfläche. Nachfolgend wird der Begriff "Axialgleitlager" synonym für beide Varianten, Axialgleitlager und Radial-Axial-Gleitlager, verwendet.

Das Motorgehäuse selbst ist mit einer geeigneten Flüssigkeit gefüllt, die einen Schmierfilm im Lagerspalt des Axialgleitlagers bildet. Alternativ kann durch eine Spülflüssigkeitszuleitung zugeführte Spülflüssigkeit, die das am distalen Ende des Motorgehäuses liegende Radiallager durchströmt, auch den Lagerspalt des Axialgleitlagers durchströmen und auf diese Weise dazu genutzt werden, den Schmierfilm im Lagerspalt zu bilden. Um in diesem Fall sicherzustellen, dass die Spülflüssigkeit das distale Radiallager mit einem Druck erreicht, der über dem anliegenden Blutdruck liegt, kann in zumindest einer der den Lagerspalt des Axialgleitlagers bildenden Oberflächen ein Kanal vorgesehen werden, der den Lagerspalt von radial außen nach radial innen durchsetzt, so dass die Spülflüssigkeit durch diesen Kanal zum distalen Radiallager fließen kann. Dieser Kanal muss nicht notwendigerweise in einer Lagerspaltoberfläche liegen, sondern kann auch als separater Kanal oder als Bohrung realisiert sein. Den Kanal in einer der Lagerspaltoberflächen vorzusehen hat aber den Vorteil, dass sich der Schmierfilm im Lagerspalt weniger erwärmt, weil ständig ein Teil des Schmierfilms durch nachströmende Spülflüssigkeit ersetzt wird. Vorzugsweise befindet sich der Kanal in der stationären Lagerspaltoberfläche, um die radiale Förderleistung zu minimieren.

Vorzugsweise ist das Axialgleitlager als hydrodynamisches Gleitlager ausgebildet. Im Gegensatz zu einem einfachen Gleitlager wird in einem hydrodynamischen Gleitlager durch Pumpwirkung der beiden relativ zueinander bewegten Oberflächen ein Druck im Schmierfilm aufgebaut. Dazu kann der Lagerspalt gemäß einer bevorzugten Variante in Umfangsrichtung des Axialgleitlagers bereichsweise als konvergierender Spalt ausgebildet sein. Dabei ist vorzugsweise die bewegte Oberfläche eben, das heißt die gegenüberliegende stationäre Oberfläche des Lagerspalts weist Rampen auf, die in Drehrichtung der bewegten Oberfläche zur bewegten Oberfläche hin konvergieren. So wird im Lagerspalt ein Keil gebildet, in den die Schmierflüssigkeit hinein transportiert wird, wodurch ein Druck aufgebaut wird, aufgrund dessen sich die bewegte Oberfläche von der statischen Oberfläche entfernt. In diesem Zustand herrscht Gleitreibung auf einem Flüssigkeitsfilm, die quasi verschleißfrei ist. Da die Blutpumpe im Normalfall kontinuierlich mit hoher Drehzahl fördert, ist die Blutpumpe besonders verschleißarm und dementsprechend für Langzeitanwendungen geeignet.

In der einfachsten Ausführungsform kann die bewegte Scheibe als Taumelscheibe ausgeführt sein und schlicht durch die Schrägstellung den kovergenten Spalt bzw. Keil bilden.

Anstelle eines Lagerspalts mit konvergierenden Oberflächenzonen kann eine der den Lagerspalt bildenden Oberflächen eine oder mehrere spiralförmig angeordnete Rillen aufweisen. In diesem Fall wird die Schmierflüssigkeit durch die Relativbewegung der beiden Oberflächen entlang der Rillen zur Lagermitte gepumpt und baut dort einen Druck auf, der wiederum dazu führt, dass sich die beiden Oberflächen voneinander entfernen. Bei der Spiralrillenlagervariante ist es bevorzugt, die spiralförmig angeordneten Rillen in der bewegten Oberfläche vorzusehen, weil so die Schmierflüssigkeitsförderung in die Rillen hinein effektiver ist.

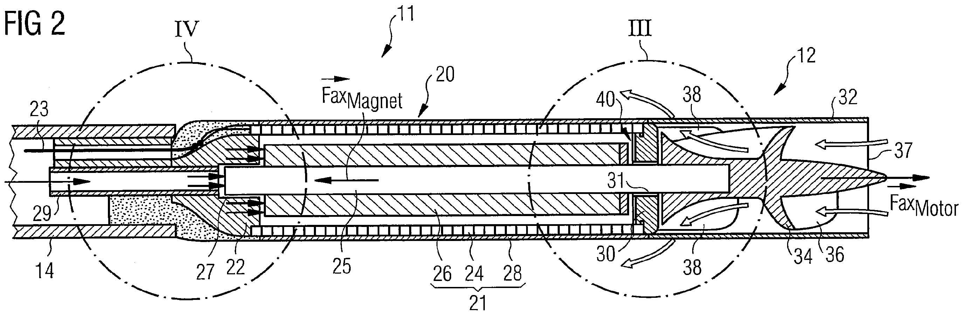

Sollte bauartbedingt der Axialschub (F_{ax Rotor}) der Pumpvorrichtung größer sein als die Tragfähigkeit des Axialgleitlagers, so kann der Axialschub der Pumpvorrichtung durch eine geeignete axiale Anordnung des rotierenden Magneten im Motor teilweise kompensiert werden. Erfindungsgemäß verhält sich der rotierende Magnet wie ein Tauchmagnet, der sich magnetisch in der Mitte des statischen Motorteils aufhalten will. Wird dieser nunmehr aus dieser Ruhelage herausgezogen, so entsteht eine Magnetkraft in umgekehrter Richtung (F_{ax Magnet}). Diese Kraft kann in Richtung des Axialschubs (F_{ax Rotor}) zur weiteren axialen Stabilisierung oder in entgegengesetzter Richtung eingesetzt werden und das axiale Gleitlager entlasten. Weiterhin kann durch Variation des Drucks der Spülflüssigkeit die resultierende Kraft auf das Axiallager eingestellt werden (vgl. Fig. 2).

Weiter bevorzugt ist es, wenn die den Lagerspalt des Axialgleitlagers bildenden Oberflächen aus Keramik bestehen, vorzugsweise Zirkoniumoxid. Aus Keramik lassen sich hochfeste, verschleißarme Oberflächen herstellen. Insbesondere kann in einfacher Weise das gesamte distale Ende des Motorgehäuses, einschließlich der Oberfläche für das Axialgleitlager, aus einem einstückigen Keramikteil hergestellt werden, so dass die Gesamtherstellungskosten der Blutpumpe gering sind.

Die Anschlussdrähte des in dem Motorgehäuse aufgenommenen Elektromotors werden normalerweise um das proximal gelegene Radiallager außen herumgeführt und mit den im Katheter verlaufenden Stromversorgungsleitungen elektrisch leitend verbunden, insbesondere verlötet. Gemäß einer bevorzugten Ausgestaltung der Erfindung werden die Anschlussdrähte des Elektromotors nun durch den Außenring des Radiallagers hindurch oder vorteilhafterweise in einer oder mehreren radial außen liegenden Nuten des Außenrings geführt. Dadurch wird Bauraum in radialer Richtung gespart, was wiederum positiv ist für die Entwicklung von Blutpumpen mit kleinem Außendurchmesser. So kann beispielsweise Bauraum für eine Druckmessung und deren Durchführung am proximalen Motorende erreicht werden.

Die Anschlussdrähte können vorteilhaft proximal von dem proximal gelegenen Radiallager auf einer Oberfläche des Motorgehäuses mit den Stromversorgungsleitungen verlötet werden. Dies ist vorteilhaft, weil das direkte elektrische Verbinden der Anschlussdrähte des Elektromotors mit den Stromversorgungsleitungen aufgrund der geringen Dicke der Anschlussdrähte und der vergleichsweise großen Dicke der Stromversorgungsleitungen Schwierigkeiten bereiten kann. Wenn die betreffende Oberfläche des Elektromotors aus einem Kunststoff oder aus Keramik besteht, wie nachfolgend noch erläutert wird, so kann der Lötstellenbereich vor dem Verlöten leitfähig beschichtet werden, beispielsweise mit Kupfer, und das Verlöten der Anschlussdrähte und der Versorgungsleitungen erfolgt jeweils separat in diesem Bereich.

Vorzugsweise wird der betreffende Teil des Motorgehäuses anschließend in Kunststoff eingebettet, wobei die Lötstellen und vorzugsweise auch die Motorwicklungen mit eingebettet werden, so dass die Lötstellen einerseits elektrisch isoliert und andererseits mechanisch geschützt sind.

Gemäß einer bevorzugten Weiterbildung der Erfindung werden auch die Radiallager für die Motorwelle am proximalen Ende und am distalen Ende des Motorgehäuses jeweils als Gleitlager ausgebildet. Da diese Radiallagerungen im wesentlichen nur dazu dienen, die Welle zentrisch genau zu führen und dementsprechend nur geringe radiale Kräfte aufzunehmen sind, können sie als einfache Gleitlager ausgeführt werden. Ein Radialgleitlager beansprucht deutlich weniger Bauraum in radialer Richtung als ein Wälzkörperlager mit seinen Innen- und Außenringen. Dies wirkt sich wieder positiv auf die Möglichkeiten aus, Blutpumpen mit kleinem Außendurchmesser herzustellen.

Insbesondere ist es bevorzugt, das am proximalen Ende des Motorgehäuses liegende Radialgleitlager aus Keramik herzustellen, wobei das Keramiklager direkt an der Umfangsoberfläche der Motorwelle anliegt. In entsprechender Weise kann auch das am distalen Ende des Motorgehäuses liegende Radiallager ausgebildet sein. Die der Keramikoberfläche gegenüberliegende Oberfläche der Motorwelle, welche zusammen mit der Keramikoberfläche den Lagerspalt des Radialgleitlagers bildet, ist vorzugsweise mit einer amorphen Kohlenstoffbeschichtung (DLC = Diamant-Like-Carbon bzw. Diamant-Like-Coating) beschichtet. DLC-Schichten sind besonders verschleißfest und reibungsarm. Sie sind nur wenige Mikrometer dick und können beispielsweise im chemischen Dampfabscheidungsverfahren (CVD) oder physikalischen Dampfabscheidungsverfahren (PVD) erzeugt werden. Alternativ kann die Welle aus einer bruchsicheren Keramik gefertigt werden.

Im Betrieb wird die Blutpumpe an eine Spülflüssigkeitsquelle angeschlossen und Flüssigkeit durch die Spülflüssigkeitsleitung hindurch ins Motorgehäuse geleitet. Die Spülflüssigkeit fließt dann durch das Axialgleitlager und weiter durch das distale Radiallager hindurch. Im Axialgleitlager bildet es den Schmierfilm im Lagerspalt. Der Druck, mit dem die Spülflüssigkeit durch das Motorgehäuse strömt, wirkt sich jedoch negativ auf die Weite des Lagerspalts aus. Denn je höher der Spülflüssigkeitsdruck ist, desto geringer wird die Lagerspaltweite und desto dünner ist der Schmierfilm zwischen den Gleitflächen. Je dünner der Schmierfilm ist, desto größer ist wiederum der zur Überwindung der Reibkräfte notwendige Motorstrom zum Antreiben des Elektromotors. Dies ist ungünstig für die Steuerung der Blutpumpe, weil man normalerweise anhand von hinterlegten Kennlinien allein auf Basis des Motorstroms und der Drehzahl (beides bekannte Größen) das aktuelle Fördervolumen ermittelt. Wenn sich der Spülflüssigkeitsdruck zusätzlich auf den Motorstrom auswirkt, so wäre eine weitere Einflussgröße zu berücksichtigen. In Anbetracht der Tatsache, dass derselbe Blutpumpentyp für unterschiedlichste Anwendungen mit unterschiedlichen Spülflüssigkeitsdrücken zwischen 300 und 1400 mmHg betrieben werden kann, ist es wichtig, eine Abhängigkeit des Motorstroms vom Spülflüssigkeitsdruck zu vermeiden.

Dies kann tatsächlich erreicht werden, wenn als Spülflüssigkeit eine Flüssigkeit mit einer Viskosität gewählt wird, die deutlich höher ist als die Viskosität von Wasser (η = 0,75 mPa • s bei 37°C). Denn mit einer hochviskosen Spülflüssigkeit wird der Flüssigkeitsfilm selbst bei hohen Drücken aufrecht erhalten und die Reibung des Axialgleitlagers ist dementsprechend unabhängig vom Spülflüssigkeitsdruck. Es hat sich herausgestellt, dass man mit einer Spülflüssigkeit, deren Viskosität bei 37°C ca. 1,2 mPa • s beträgt oder noch darüber liegt, das Axialgleitlager als einfaches Gleitlager ausbilden kann und nicht als hydrodynamisches Gleitlager auszubilden braucht. Gute Ergebnisse wurden beispielsweise mit einer ≥ 20%-igen Glykoselösung zwischen Keramikoberflächen aus Zirkoniumoxid erzielt.

Nachfolgend wird die Erfindung beispielhaft anhand der begleitenden Zeichnungen erläutert. Darin zeigen:
- Figur 1: eine schematische Darstellung der Einführung einer Blutpumpe vor den linken Ventrikel, mit Positionierung ihrer Ansaugkanüle im linken Ventrikel,
- Figur 2: einen schematischen Längsschnitt eines Ausführungsbeispiels der Blutpumpe,
- Figur 3: eine vergrößerte Darstellung der Einzelheit III aus Figur 2,
- Figur 4: eine Variante zur Einzelheit III aus Figur 3,
- Figur 5: eine vergrößerte Darstellung der Einzelheit IV aus Figur 2,
- Figuren 6A und 6B: eine Axialgleitlageroberfläche in Draufsicht und als Abwicklung gemäß einem ersten Ausführungsbeispiel,
- Figur 7: eine Axialgleitlageroberfläche im Querschnitt gemäß einem zweiten Ausführungsbeispiel, und
- Figur 8: eine Axialgleitlageroberfläche in Draufsicht gemäß einem dritten Ausführungsbeispiel.

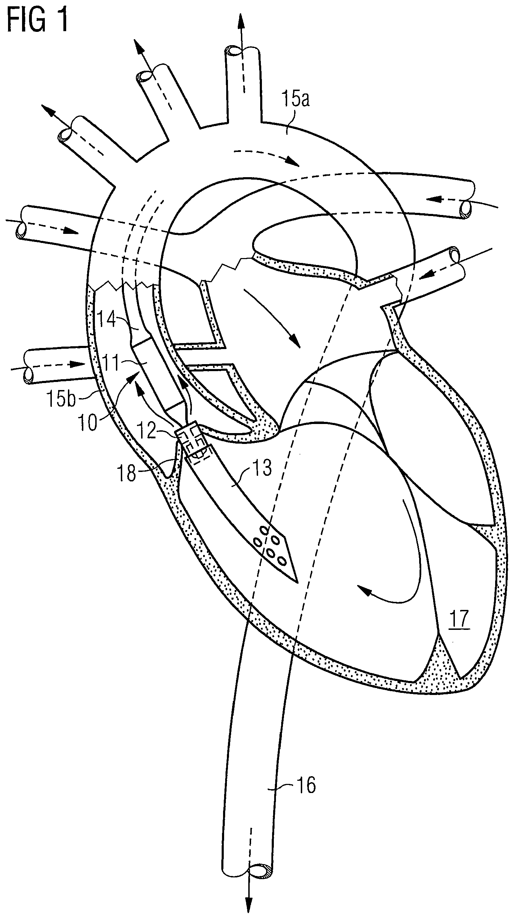

In Figur 1 ist die Verwendung einer Blutpumpe 10 zur Unterstützung des linken Ventrikels dargestellt. Die Blutpumpe weist einen Motorteil 11 und einen Pumpenteil 12 auf, die koaxial hintereinander angeordnet sind und eine stabförmige Bauform ergeben. Der Pumpenteil ist durch einen flexiblen Saugschlauch 13 verlängert, der an seinem Ende und/oder in seiner Seitenwand Öffnungen für den Blutzutritt zur Pumpe aufweist. Das vom Saugschlauch 13 abgewandte Ende der Blutpumpe 10 ist mit einem Katheter 14 verbunden, der durch den Aortenbogen 15a und die Aorta 16 eingeführt wurde. Die Blutpumpe 10 wird so platziert, dass sie hauptsächlich in der aufsteigenden Aorta 15b liegt, der Pumpenteil 12 mit dem Saugschlauch 13 aber im wesentlichen im linken Ventrikel 17 liegt. Die Aortenklappe 18 legt sich im Schließzustand an die Außenseite des Pumpengehäuses oder des Saugschlauchs 13 an. Die Blutpumpe 10 mit dem vorgelagerten Saugschlauch 13 wird durch Vorschieben des Katheters 14 gegebenenfalls unter Verwendung eines Führungsdrahts in die dargestellte Position vorgeschoben. Der Saugschlauch 13 passiert dabei die Aortenklappe 18 retrograd, so dass durch den Saugschlauch 13 Blut angesaugt und in die Aorta 16 gepumpt wird. Insoweit entspricht die Blutpumpe der aus der EP 0 961 621 B1 bekannten Blutpumpe.

Der Einsatz der Blutpumpe ist nicht auf die in Figur 1 dargestellte Anwendung beschränkt, bei der es sich lediglich um ein typisches Anwendungsbeispiel handelt. So kann die Pumpe auch durch andere periphere Gefäße, wie die Arteria Subclavia, eingeführt oder auch im rechen Herzen platziert werden.

Figur 2 zeigt ein bevorzugtes Ausführungsbeispiel der Blutpumpe mit dem Motorteil 11 und dem damit fest verbundenen Pumpenteil 12. Der Motorteil 11 weist ein langgestrecktes Gehäuse 20 auf, in welchem der Elektromotor 21 untergebracht ist. Der Stator 24 des Elektromotors 21 weist in üblicher Weise zahlreiche umfangsmäßig verteilt angeordnete Wicklungen sowie einen magnetischen Rückschluss 28 in Längsrichtung auf. Er ist mit dem Motorgehäuse fest verbunden. Der Stator 24 umgibt den mit der Motorwelle 25 verbundenen Rotor 26, der aus in Wirkrichtung magnetisierten Permanentmagneten besteht. Die Motorwelle 25 erstreckt sich über die gesamte Länge des Motorgehäuses 20 und ragt distal aus diesem heraus. Dort trägt sie ein Flügelrad 34 mit davon abstehenden Flügeln 36 oder Pumpenschaufeln, die in einem rohrförmigen Pumpengehäuse 32 drehen, welches seinerseits fest mit dem Motorgehäuse 20 verbunden ist.

An das proximale Ende des Motorgehäuses 20 schließt sich der flexible Katheter 14 abdichtend an. Durch den Katheter 14 verlaufen elektrische Kabel 23 zur Stromversorgung und Steuerung des Elektromotors 21. Desweiteren verläuft durch den Katheter 14 eine Spülflüssigkeitsleitung 29, welche die proximale Stirnwand 22 des Motorgehäuses 20 durchsetzt. Spülflüssigkeit wird durch die Spülflüssigkeitsleitung 29 ins Innere des Motorgehäuses 20 gespeist und tritt durch die Stirnseite 30 am distalen Ende des Motorgehäuses aus. Der Spüldruck ist so gewählt, dass er über dem anliegenden Blutdruck liegt, um auf diese Weise zu verhindern, dass Blut ins Motorgehäuse eindringt, und liegt je nach Anwendungsfall zwischen 300 und 1400 mmHg.

Bei einer Rotation des Flügelrades 34 wird Blut durch die stirnseitige Ansaugöffnung 37 des Pumpengehäuses 32 angesaugt und in axialer Richtung im Pumpengehäuse 32 nach hinten befördert. Durch Austrittsöffnungen 38 im Pumpengehäuse 32 strömt das Blut aus dem Pumpenteil 12 aus und weiter entlang dem Motorgehäuse 20. Hierdurch wird der Abtransport der im Antrieb erzeugten Wärme sichergestellt. Es ist auch möglich, den Pumpenteil mit umgekehrter Förderrichtung zu betreiben, wobei Blut an dem Motorgehäuse 20 entlang angesaugt wird und aus der Öffnung 37 austritt.

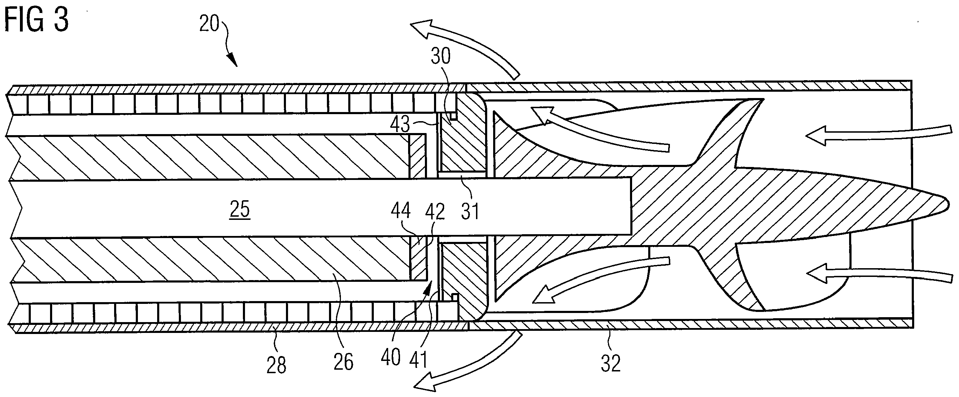

Die Motorwelle 25 ist einerseits am proximalen Ende des Motorgehäuses und andererseits am distalen Ende des Motorgehäuses in Radiallagern 27 und 31 gelagert. Die Radiallager sind in diesem Ausführungsbeispiel jeweils als einfache Gleitlager ausgeführt. Darüber hinaus ist die Motorwelle 25 in dem Motorgehäuse 20 auch axial gelagert. Das Axiallager 40 ist ebenfalls als Gleitlager ausgeführt. Das Axialgleitlager 40 wird nachfolgend in Bezug auf Figur 3 genauer erläutert. Es dient dazu, axiale Kräfte der Motorwelle 25, die in distaler Richtung wirken, wenn das Flügelrad 34 von distal nach proximal fördert, aufzunehmen. Sollte die Blutpumpe dazu eingesetzt werden, Blut auch oder nur in umgekehrter Richtung zu fördern, so ist ein entsprechendes Axialgleitlager 40 (auch/oder) in entsprechender Weise am proximalen Ende des Motorgehäuses 20 vorzusehen.

Die Blutpumpe gemäß Fig. 2 kann alternativ ohne Spülflüssigkeit für den kurzzeitigen Einsatz von wenigen Stunden verwendet werden. Für diesen Fall werden die Gleitlager einmalig geschmiert, und das distale Gleitlager 31 ist zudem mit einer Radiallippendichtung versehen, um den Eintritt von Blut zu verhindern. Auf eine Spülflüssigkeitsleitung kann dann vorteilhaft insgesamt verzichtet werden.

Figur 3 zeigt den Ausschnitt III aus Figur 2 in größerem Detail. Zu sehen sind insbesondere das Radialgleitlager 31 und das Axialgleitlager 40. Der Lagerspalt des Radialgleitlagers 31 wird gebildet einerseits durch die Umfangsoberfläche der Motorwelle 25, die DLC-beschichtet ist, und andererseits durch die Oberfläche der Durchgangsbohrung in der distalen Stirnwand 30 des Motorgehäuses 20, welche als Keramikteil, zum Beispiel aus Zirkoniumoxid, hergestellt ist.

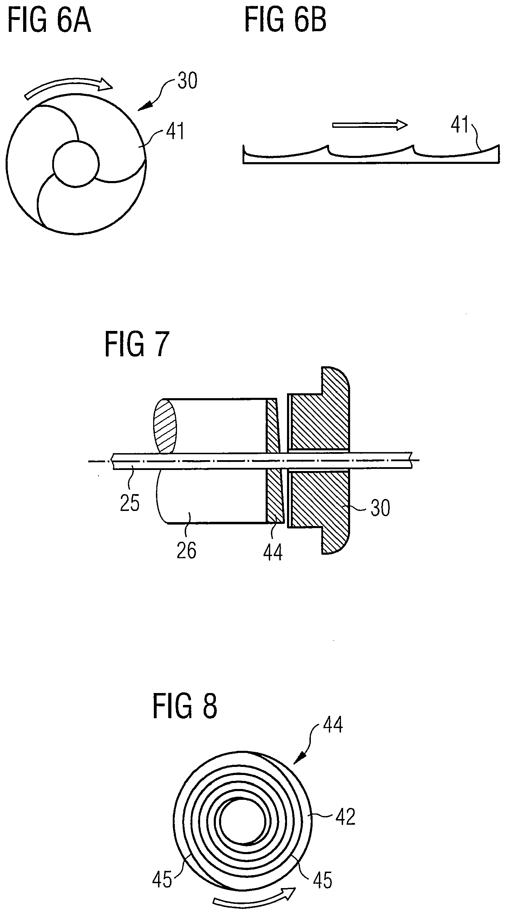

Der Lagerspalt des Axialgleitlagers 40 wird einerseits durch die axial innen liegende Oberfläche 41 der Stirnwand 30 und eine ihr gegenüberliegende Oberfläche 42 gebildet. Diese gegenüberliegende Oberfläche 42 ist Bestandteil einer Keramikscheibe 44, die auf der Motorwelle 25 distal vom Rotor 26 sitzt und sich mit dem Rotor 26 dreht. Ein Kanal 43 in der Lagerspaltoberfläche 41 der Stirnwand 30 stellt sicher, dass Spülflüssigkeit zwischen den Lagerspaltoberflächen 41 und 42 des Axialgleitlagers 40 hindurch zum Radialgleitlager 31 fließen und distal aus dem Motorgehäuse 20 austreten kann. Die Spülflüssigkeit wird mit einer Viskosität von mindestens 1,2 mPa • s bei 37°C gewählt. Als geeignet hat sich beispielsweise 20%-Glykoselösung erwiesen. Das in Figur 3 dargestellte Axialgleitlager 40 ist ein normales Gleitlager. Hydrostatische Gleitlagervarianten werden nachfolgend in Bezug auf die Figuren 6A/B, 7 und 8 beschrieben. Der Axialspalt des Axialgleitlagers 40 ist anders als dargestellt mit wenigen µm sehr klein.

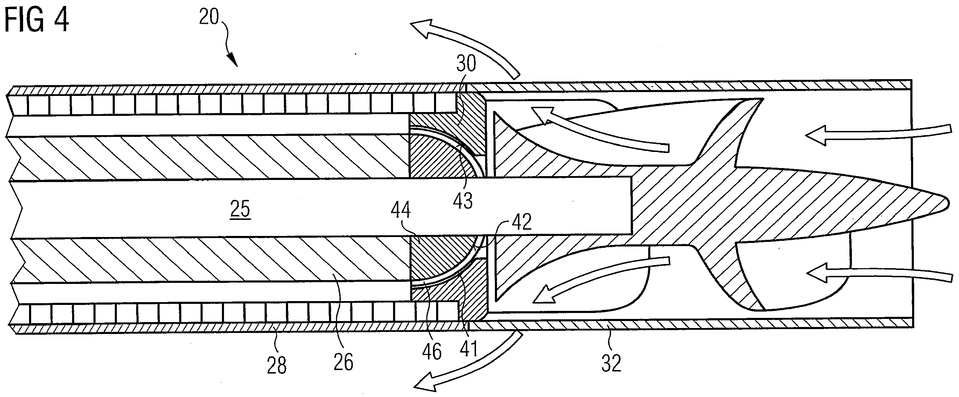

Anstelle des Axialgleitlagers 40 und Radialgleitlagers 31 kann auch ein kombiniertes Radial-Axial-Gleitlager 46 mit einer konkaven Lagerschale, in der eine konvexe Lagerfläche läuft, realisiert werden. Eine solche Variante ist in Figur 4 anhand eines sphärischen Gleitlagers 46 dargestellt. Die Lagerspaltoberfläche 41 ist sphärisch konkav gestaltet, und die gegenüberliegende Lagerspaltoberfläche 42 ist entsprechend sphärisch konvex ausgebildet. Der Kanal 43 liegt wieder in der ortsfesten Lagerspaltoberfläche 41 der Stirnwand 30. Alternativ kann die feststehende Lagerspaltoberfläche 41 der Stirnwand 30 konvex und die gegenüberliegende Lagerspaltoberfläche 42 konkav ausgebildet werden. Die Oberflächen 42, 43 können anstatt sphärisch auch konisch sein. Vorzugsweise ist ein entsprechendes Radial-Axial-Gleitlager an beiden Seiten des Motorgehäuses 20 vorgesehen, um bei axialem Wandern der Welle 25 keinen radialen Versatz zuzulassen. Der Vorteil eines kombinierten Axial-Radial-Gleitlagers liegt in der höheren Belastbarkeit. Nachteilig ist hingegen der größere Reibdurchmesser.

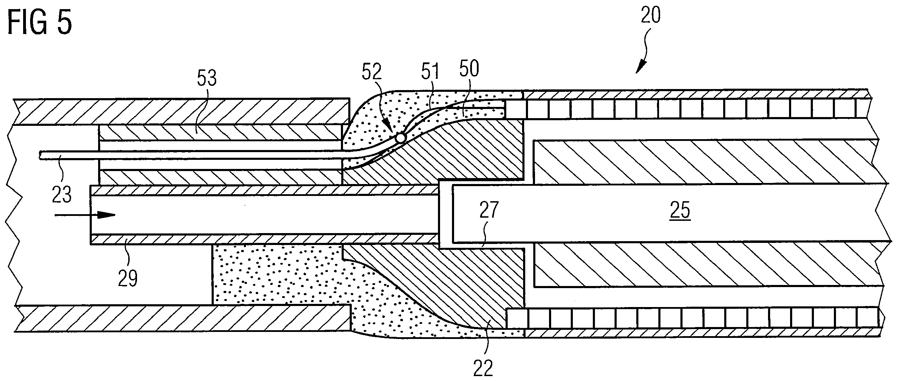

Figur 5 zeigt das Radiallager 27 am distalen Ende des Motorgehäuses 20. Auch hier ist die Motorwelle 25 mit einer DLC-Beschichtung versehen und läuft in einer Lagerbuchse, die einen integralen Bestandteil der wiederum aus Keramik gefertigten proximalen Stirnwand 22 des Motorgehäuses 20 bildet. Insofern entspricht das Radialgleitlager 27 dem Radialgleitlager 31.

Über den Umfang der Stirnwand 22 verteilt sind drei axial verlaufende Nuten 50 im Abstand von 120° vorgesehen, von denen in Figur 2 lediglich eine zu sehen ist. Durch diese Nuten 50 hindurch führen dünne Anschlussdrähte 51 zu den Wicklungen des Stators 24. Die Anschlussdrähte 51 werden auf der proximalen Seite der Stirnwand 20 festgelötet, wobei die Lötstelle 52 zuvor mit einer lokalen Kupferbeschichtung leitfähig gemacht wird. An derselben Lötstelle 52 wird außerdem das Ende der Stromversorgungsleitung 23 verlötet. Die Anbindung der Drähte der Statorwicklungen mit den Stromversorgungsleitungen kann mit allen herkömmlichen Fügeverfahren (Löten, Schweißen, Klemmen, Laserschweißen, Spaltschweißen, Kontaktkleben, etc.) erfolgen. Anschließend wird die Stirnwand 22 einschließlich der Anschlussdrähte 51 und der Lötstellen 52 mit einem Kunststoffmaterial ummantelt, wobei gleichzeitig auch die Motorwicklungen des Stators mit ummantelt werden. Dies kann beispielhaft in einem Vakuumverguß erfolgen.

Die vorbeschriebene Blutpumpe verzichtet auf Radialkugellager zur Lagerung der Motorwelle 25, welche schwer zu montieren sind und eine Mindestgröße von 3 mm besitzen. Dadurch ist es möglich, Pumpen mit noch kleineren Außendurchmessern von beispielsweise lediglich 3 mm herzustellen. Außerdem ist die Lebensdauer dieser Blutpumpe gegenüber solchen mit Radialkugellagern aufgrund geringeren Verschleißes deutlich erhöht. Laufzeiten > 30 Tage können so verschleißarm realisiert werden. Letzteres ist äußerst bedeutsam, da die Lagerung und der schlagfreie Lauf des Flügelrades entscheidend sind für eine geringe Blutschädigung.

Figur 6A zeigt in Aufsicht die Oberfläche 41 der distalen Stirnwand 30 des Motorgehäuses 20 gemäß einem alternativen Ausführungsbeispiel. Figur 6B zeigt eine Abwicklung der Oberfläche 41 aus Figur 6A. Die Oberfläche 41 selbst ist stationär. Die durch den Pfeil angedeutete Richtung zeigt an, in welcher Richtung sich die gegenüberliegende Oberfläche 42 des Gleitlagers 40 bewegt. Dies entspricht dann auch der Richtung, in der sich der Schmierfilm innerhalb des Lagerspalts relativ zur stationären Oberfläche 41 bewegt. Dementsprechend besitzt die Oberfläche 41 hintereinander angeordnete Rampen, die zusammen mit der gegenüberliegenden bewegten Oberfläche 42, welche eben ist, konvergierende Spalte bilden. Dadurch baut sich im Schmierfilm ein hydrodynamischer Druck auf, der dafür sorgt, dass die den Lagerspalt bildenden Oberflächen auf Abstand bleiben.

Eine Ausbildung der rotierenden Oberfläche mit den rampenartigen Strukturen gemäß den Figuren 6A und 6B ist zwar für die Leistungsfähigkeit des axialen Gleitlagers von Vorteil, führt aber zu einer erhöhten radialen Förderwirkung im Lagerspalt, die der Förderrichtung der Spülflüssigkeit entgegensteht. In Figur 7 ist die einfachste Form einer rampenartigen Realisierung des konvergenten Spalts in Form einer Taumelscheibe dargestellt. Hierbei wird einfach die Scheibe 44 schrägt eingebaut oder minimal schräg geschliffen. Die Schrägstellung beträgt typischerweise 1 bis 5 µm.

Figur 8 zeigt eine andere Variante für eine hydrodynamisch wirkende Oberfläche des Axialgleitlagers 40. Es handelt sich um ein sogenanntes Spiralrillenlager, welches bevorzugt an der sich bewegenden Oberfläche des Lagerspalts ausgebildet wird, also dementsprechend an der Oberfläche 42 der Keramikscheibe 44. In diesem Falle sind in der Oberfläche 42 mehrere Rillen 45 spiralförmig angeordnet. Die Rillen 45 sind in Figur 8 lediglich schematisch angedeutet. Wenn die Keramikscheibe 44 in der durch den Pfeil in Figur 8 angedeuteten Richtung dreht, so wird der Schmierfilm entlang der Rillen 45 nach radial innen gefördert und baut dort einen Druck auf, der wiederum dafür sorgt, dass die den Lagerspalt bildenden Oberflächen zueinander auf Abstand gehalten werden.

## Patentansprüche

1. Intravasale Blutpumpe (10), umfassend
- einen Antriebsteil (11), der ein Motorgehäuse (20) mit einem proximalen Ende und einem distalen Ende und einen in dem Motorgehäuse angeordneten Elektromotor (21) aufweist, wobei der Elektromotor eine Motorwelle (25) besitzt, die mit einem Ende aus dem distalen Ende des Motorgehäuses (20) herausragt und in dem Motorgehäuse sowohl am proximalen Ende als auch am distalen Ende des Motorgehäuses radial gelagert (27, 31) ist, wobei die Motorwelle (25) mittels mindestens einem Axialgleitlager (40) oder Radial-Axial-Gleitlager (46) axial in dem Motorgehäuse (20) gelagert ist,
- einen Katheter (14), der mit dem proximalen Ende des Motorgehäuses (20) verbunden ist und entlang dessen Leitungen (23) zur Stromversorgung des Elektromotors verlaufen, und
- einen Pumpenteil (12) mit einem rohrförmigen Pumpengehäuse (32), das am distalen Ende des Motorgehäuses (20) befestigt ist, und einem Flügelrad (34), das auf dem aus dem distalen Ende des Motorgehäuses herausragenden Ende der Motorwelle (25) sitzt und in dem Pumpengehäuse (32) drehbar ist,
**dadurch gekennzeichnet, dass** der Motor (21) einen statischen Motorteil (24) und einen rotierenden Motorteil (26) aufweist, wobei der rotierende Motorteil (26) als Tauchmagnet ausgebildet ist, den es magnetisch in eine Ruhelage in die Mitte des statischen Motorteils (24) drängt, wobei der Tauchmagnet aus dieser Ruhelage axial herausgezogen angeordnet ist.

2. Intravasale Blutpumpe nach Anspruch 1, wobei das Axialgleitlager (40) oder Radial-Axial-Gleitlager (46) eine auf der Motorwelle (25) angeordnete Scheibe (44) umfasst, die sich gegen eine umlaufende Schulter des Motorgehäuses (20) abstützt.

3. Intravasale Blutpumpe nach einem der Ansprüche 1 oder 2, umfassend eine Spülflüssigkeitsleitung (29), die so angeordnet ist, dass durch die Spülflüssigkeitsleitung zugeführte Flüssigkeit den Lagerspalt des Axialgleitlagers (40) und das am distalen Ende des Motorgehäuses liegende Radiallager (31) bzw. den Lagerspalt des Radial-Axial-Gleitlagers (46) durchströmt.

4. Intravasale Blutpumpe nach einem der Ansprüche 1 bis 3, wobei zumindest eine der den Lagerspalt des Axialgleitlagers (40) oder Radial-Axial-Gleitlagers (46) bildenden Oberflächen (41, 42) einen Kanal (43) aufweist, der den Lagerspalt von radial außen nach radial innen durchsetzt.

5. Intravasale Blutpumpe nach einem der Ansprüche 1 bis 4, wobei der Lagerspalt des Axialgleitlagers (40) oder Radial-Axial-Gleitlagers (46) in Umfangsrichtung bereichsweise als konvergierender Spalt ausgebildet ist.

6. Intravasale Blutpumpe nach Anspruch 5, wobei eine bewegte Oberfläche (42) der den Lagerspalt des Axialgleitlagers (40) oder Radial-Axial-Gleitlagers (46) bildenden Oberflächen (42, 41) eben ist.

7. Intravasale Blutpumpe nach einem der Ansprüche 1 bis 4, wobei eine der den Lagerspalt des Axialgleitlagers (40) oder Radial-Axial-Gleitlagers (46) bildenden Oberflächen (41, 42) eine oder mehrere spiralförmig angeordnete Rillen (45) aufweist.

8. Intravasale Blutpumpe nach Anspruch 7, wobei eine bewegte Oberfläche (42) der den Lagerspalt des Axialgleitlagers (40) oder Radial-Axial-Gleitlagers (46) bildenden Oberflächen (41 , 42) die spiralförmig angeordnete(n) Rille(n) aufweist.

9. Intravasale Blutpumpe nach einem der Ansprüche 1 bis 8, wobei die den Lagerspalt des Axialgleitlagers (40) oder Radial-Axial-Gleitlagers (46) bildenden Oberflächen (41 , 42) aus Keramik bestehen.

10. Intravasale Blutpumpe nach einem der Ansprüche 1 bis 9, wobei das am proximalen Ende des Motorgehäuses (20) gelegene Radiallager (27) einen Außenring aufweist und Anschlussdrähte (51) des Elektromotors (21) durch den Außenring hindurch oder in einer radial außen liegenden Nut (50) des Außenrings verlaufen.

11. Intravasale Blutpumpe nach Anspruch 10, wobei die Anschlussdrähte (51) des Elektromotors (21) und die entlang dem Katheter (14) verlaufenden Leitungen (23) auf einer proximal von dem am proximalen Ende des Motorgehäuses gelegenen Radiallager (27) liegenden Oberfläche (52) elektrisch leitend verbunden sind.

12. Intravasale Blutpumpe nach Anspruch 11, wobei das Motorgehäuse zumindest teilweise ein gegossenes Kunststoffgehäuse ist, und wobei die Verlötungen (52) mit umgossen sind.

13. Intravasale Blutpumpe nach einem der Ansprüche 1 bis 9, wobei zumindest eines der radialen Lagerungen (27, 31) der Motorwelle (25) am proximalen Ende und am distalen Ende des Motorgehäuses (20) als Radialgleitlager ausgebildet sind.

14. Intravasale Blutpumpe nach Anspruch 13, wobei bei dem zumindest einen Radialgleitlager eine der den Lagerspalt bildenden Oberflächen eine Keramikoberfläche und die entsprechend andere der den Lagerspalt bildenden Oberflächen eine amorphe Kohlenstoffbeschichtung (DLC) ist.

15. System, umfassend die intravasale Blutpumpe nach Anspruch 3 und eine Spülflüssigkeitsquelle zur Versorgung der Spülflüssigkeitsleitung mit einer Flüssigkeit, deren Viskosität bei 37°C ≥ 1,2 mPa • s liegt.

## Claims

1. An intravascular blood pump (10), comprising
- a drive section (11) which has a motor housing (20) having a proximal end and a distal end and an electric motor (21) disposed in the motor housing, with the electric motor possessing a motor shaft (25) which protrudes at one end out of the distal end of the motor housing (20) and is mounted radially (27, 31) in the motor housing both at the proximal end and at the distal end of the motor housing, with the motor shaft (25) being mounted in the motor housing (20) axially by means of at least one axial sliding bearing (40) or radial-axial sliding bearing (46),
- a catheter (14) which is connected to the proximal end of the motor housing (20) and along which there extend lines (23) for power supply to the electric motor, and
- a pump section (12) having a tubular pump housing (32) which is fastened to the distal end of the motor housing (20), and an impeller (34) which is seated on the end of the motor shaft (25) protruding out of the distal end of the motor housing and is rotatable in the pump housing (32),
**characterized in that** the motor (21) has a static motor section (24) and a rotating motor section (26), with the rotating motor section (26) being configured as a solenoid which is displaced magnetically into the center of the static motor section (24) into a rest position, with the solenoid being disposed so as to be pulled out of said rest position axially.

2. The intravascular blood pump according to claim 1, wherein the axial sliding bearing (40) or radial-axial sliding bearing (46) comprises a disk (44) disposed on the motor shaft (25) and supported against a circumferential shoulder of the motor housing (20).

3. The intravascular blood pump according to either of claims 1 and 2, comprising a purge-fluid line (29) which is so disposed that fluid fed through the purge-fluid line flows through the bearing gap of the axial sliding bearing (40) and the radial bearing (31) located at the distal end of the motor housing, or the bearing gap of the radial-axial sliding bearing (46).

4. The intravascular blood pump according to any of claims 1 to 3, wherein at least one of the surfaces (41, 42) forming the bearing gap of the axial sliding bearing (40) or radial-axial sliding bearing (46) has a channel (43) which penetrates the bearing gap from radially outward to radially inward.

5. The intravascular blood pump according to any of claims 1 to 4, wherein the bearing gap of the axial sliding bearing (40) or radial-axial sliding bearing (46) is configured as a converging gap in some regions in the circumferential direction.

6. The intravascular blood pump according to claim 5, wherein a moved surface (42) of the surfaces (42, 41) forming the bearing gap of the axial sliding bearing (40) or radial-axial sliding bearing (46) is even.

7. The intravascular blood pump according to any of claims 1 to 4, wherein one of the surfaces (41, 42) forming the bearing gap of the axial sliding bearing (40) or radial-axial sliding bearing (46) has one or more spirally disposed grooves (45).

8. The intravascular blood pump according to claim 7, wherein a moved surface (42) of the surfaces (41, 42) forming the bearing gap of the axial sliding bearing (40) or radial-axial sliding bearing (46) has the spirally disposed groove(s).

9. The intravascular blood pump according to any of claims 1 to 8, wherein the surfaces (41, 42) forming the bearing gap of the axial sliding bearing (40) or radial-axial sliding bearing (46) are made of ceramic.

10. The intravascular blood pump according to any of claims 1 to 9, wherein the radial bearing (27) situated at the proximal end of the motor housing (20) has an outer ring, and lead wires (51) of the electric motor (21) extend through the outer ring or within a radially outwardly located slot (50) of the outer ring.

11. The intravascular blood pump according to claim 10, wherein the lead wires (51) of the electric motor (21) and the lines (23) extending along the catheter (14) are connected electroconductively on a surface (52) located proximally of the radial bearing (27) situated at the proximal end of the motor housing.

12. The intravascular blood pump according to claim 11, wherein the motor housing is at least partly a cast plastic housing, and wherein the solderings (52) are cast in as well.

13. The intravascular blood pump according to any of claims 1 to 9, wherein at least one of the radial bearings (27, 31) of the motor shaft (25) is configured as a radial sliding bearing at the proximal end and at the distal end of the motor housing (20).

14. The intravascular blood pump according to claim 13, wherein, in the at least one radial sliding bearing, one of the surfaces forming the bearing gap is a ceramic surface and the corresponding other one of the surfaces forming the bearing gap is an amorphous carbon coating (DLC).

15. A system comprising the intravascular blood pump according to claim 3 and a purge-fluid source for supplying the purge-fluid line with a fluid whose viscosity at 37°C lies ≥ 1.2 mPa • s.

## Revendications

1. Pompe à sang (10) intravasculaire comprenant
- une partie entraînement (11) munie d'un boîtier de moteur (20) ayant une extrémité proximale et une extrémité distale et d'un électromoteur (21) agencé dans le boîtier de moteur, l'électromoteur possédant un arbre moteur (25) qui, par une extrémité, dépasse de l'extrémité distale du boîtier de moteur (20) et qui, dans le boîtier de moteur, est logé radialement (27, 31) tant à l'extrémité proximale qu'à l'extrémité distale du boîtier de moteur, l'arbre moteur (25) étant logé axialement dans le boîtier de moteur (20) au moyen d'au moins un palier lisse axial (40) ou palier lisse radial-axial (46),
- un cathéter (14) qui est relié à une extrémité proximale du boîtier de moteur (20) et le long duquel passent des conduites (23) destinées à l'alimentation électrique de l'électromoteur, et
- une partie pompe (12) munie d'un boîtier de pompe (32) de forme tubulaire fixé à l'extrémité distale du boîtier de moteur (20) et d'une roue à ailettes (34) qui est située l'extrémité de l'arbre moteur (25) dépassant de l'extrémité distale du boîtier de moteur et qui peut tourner dans le boîtier de pompe (32),
**caractérisée en ce que** le moteur (21) comporte une partie statique de moteur (24) et une partie tournante de moteur (26), la partie tournante de moteur (26) étant réalisée sous forme d'aimant plongeant qui est magnétiquement poussé à adopter une position de repos au centre de la partie statique de moteur (24), l'aimant plongeant étant agencé de telle manière qu'il est retiré axialement de cette position de repos.

2. Pompe à sang intravasculaire selon la revendication 1, le palier lisse axial (40) ou palier lisse radial-axial (46) comprenant un disque (44) qui est agencé sur l'arbre moteur (25) et s'appuie contre un épaulement circulaire du boîtier de moteur (20).

3. Pompe à sang intravasculaire selon une des revendications 1 ou 2, comprenant une conduite de liquide de rinçage (29) agencée de telle façon que du liquide amené par la conduite de liquide de rinçage s'écoule à travers la fente de palier du palier lisse axial (40) et à travers le palier radial (31) situé à l'extrémité distale du boîtier de moteur ou à travers la fente de palier du palier lisse radial-axial (46).

4. Pompe à sang intravasculaire selon une des revendications de 1 à 3, cependant qu'au moins une des surfaces (41, 42) constituant la fente de palier du palier lisse axial (40) ou du palier lisse radial-axial (46) comporte un canal (43) qui traverse la fente de palier depuis radialement l'extérieur vers radialement l'intérieur.

5. Pompe à sang intravasculaire selon une des revendications de 1 à 4, la fente de palier du palier lisse axial (40) ou du palier lisse radial-axial (46) étant réalisée, dans le sens périphérique, par zones sous forme d'une fente convergente.

6. Pompe à sang intravasculaire selon la revendication 5, une surface mobile (42) des surfaces (42, 41) constituant la fente de palier du palier lisse axial (40) ou du palier lisse radial-axial (46) étant plane.

7. Pompe à sang intravasculaire selon une des revendications de 1 à 4, une des surfaces (41, 42) constituant la fente de palier du palier lisse axial (40) ou du palier lisse radial-axial (46) comportant une ou plusieurs cannelures (45) agencées en spirale.

8. Pompe à sang intravasculaire selon la revendication 7, une surface mobile (42) des surfaces (41, 42) constituant la fente de palier du palier lisse axial (40) ou du palier lisse radial-axial (46) comportant la/les cannelure(s) agencée(s) en spirale.

9. Pompe à sang intravasculaire selon une des revendications de 1 à 8, les surfaces (42, 41) constituant la fente de palier du palier lisse axial (40) ou du palier lisse radial-axial (46) étant en céramique.

10. Pompe à sang intravasculaire selon une des revendications de 1 à 9, le palier radial (27) situé à l'extrémité proximale du boîtier de moteur (20) comportant une bague extérieure, et des fils de raccordement (51) de l'électromoteur (21) passant à travers la bague extérieure ou dans une rainure (50) radialement extérieure de la bague extérieure.

11. Pompe à sang intravasculaire selon la revendication 10, les fils de raccordement (51) de l'électromoteur (21) et les conduites (23) passant le long du cathéter (14) étant reliés de manière électroconductrice sur une surface (52) proximale du palier radial (27) situé à l'extrémité proximale du boîtier de moteur.

12. Pompe à sang intravasculaire selon la revendication 11, le boîtier de moteur étant au moins partiellement un boîtier en matière plastique moulé, et les soudures (52) étant surmoulées en même temps.

13. Pompe à sang intravasculaire selon une des revendications de 1 à 9, au moins un des logements radiaux (27, 31) de l'arbre moteur (25) à l'extrémité proximale et à l'extrémité distale du boîtier de moteur (20) étant réalisés sous forme de paliers lisses radiaux.

14. Pompe à sang intravasculaire selon la revendication 13, cependant que, dans le au moins un palier lisse radial, une des surfaces constituant la fente de palier est une surface céramique, et l'autre surface correspondante des surfaces constituant la fente de palier est un revêtement de carbone amorphe (DLC).

15. Système comprenant la pompe à sang intravasculaire selon la revendication 3 et une source de liquide de rinçage destinée à l'alimentation de la conduite de liquide de rinçage en un liquide dont la viscosité à 37° C est ≤ 1,2 mPa · s.
